# EUROPEAN PATENT SPECIFICATION

(11) **EP 1 356 825 B1**
(45) Date of publication and mention of the grant of the patent: **02.11.2005**
(21) Application number: 02710419.9
(22) Date of filing: 31.01.2002
(51) Int. Cl.: C08B 15/04, C08J 3/07, A61K 47/38, A61K 9/28

(54) **PROCESS FOR PRODUCING AQUEOUS CELLULOSE DERIVATIVE DISPERSION**
VERFAHREN ZUR HERSTELLUNG EINER WÄSSRIGEN ZELLULOSEDERIVAT-DISPERSION
PROCEDE DE PRODUCTION D'UNE DISPERSION AQUEUSE DE DERIVES CELLULOSIQUES

(30) Priority: 31.01.2001 JP 2001023796
(43) Date of publication of application: 29.10.2003
(73) Proprietor: Asahi Kasei Chemicals Corporation, Tokyo 100-8440 (JP)
(72) Inventor: KAMADA, Etsuo, Nobeoka-shi, Miyazaki 882-0003 (JP); TANOUCHI, Masatoshi, Nobeoka-shi, Miyazaki 882-0847 (JP); NAGATOMO, Sueo, Nobeoka-shi, Miyazaki 882-0016 (JP)
(74) Representative: Weber, Thomas
(86) International application number: PCT/JP2002/000761
(87) International publication number: WO 2002/060487

(56) References cited:
- EP-A- 0 662 323
- EP-A1- 0 541 369
- EP-A2- 0 136 722
- JP-A- 60 031 501

## Description

### TECHNICAL FIELD

The present invention relates to a method for producing a cellulose derivative aqueous dispersion, an aqueous dispersion obtained by the method, and a coated pharmaceutical(s) obtained by using the aqueous dispersion. More particularly, it relates to a method for producing a cellulose derivative aqueous dispersion excellent in film-formability and resistance to a first liquid, as well as an aqueous dispersion obtained by the method. Furthermore, it relates to an enteric pharmaceutical(s) produced by an aqueous process using the aqueous dispersion as a coating material for foods or pharmaceutical(s).

### BACKGROUND ART

Cellulose derivatives having carboxyl groups are widely utilized for cosmetics, pharmaceuticals, agricultural chemicals, etc. Especially, they are used as enteric coating bases in the field of pharmaceuticals.

As enteric coating agents, although organic solvent-based agents have been mainly used, the conventionally used organic solvent-based agents are gradually substituted for aqueous agents due to the residual organic solvent in the pharmaceutical(s), high cost of the organic solvents, and for improvement of working atmosphere. Hitherto, there have been proposed as a method for producing an enteric cellulose derivative aqueous coating agent, a method which comprises dissolving an enteric cellulose derivative in an organic solvent, finely dispersing the solution in water in the presence of an emulsifier, and then removing the organic solvent from the dispersion to produce solid particles of the enteric cellulose derivative in the aqueous phase (JP-B-2-7925); a method which comprises directly dispersing fine powders of carboxymethyl cellulose in water (JP-A-56-154420); a method which comprises dissolving carboxymethylethyl cellulose in an aqueous alcohol solution, adding various additives such as a plasticizer, and then removing the alcohol to obtain a latex (JP-A-61-207342); a method for producing cellulose derivative latex which comprises dissolving a cellulose derivative having an anionic functional group into an organic solvent immiscible with water, dispersing the solution in water in the presence of an alkali and removing the organic solvent from the resulting dispersion (JP-A-5-125224); a method wherein an alkylation degree, a carboxyalkylation degree, a ratio of carboxylic acid group and carboxylic acid salt group, and a ratio of carboxylic acid salt group and alkylation degree are specified in an aqueous enteric coating agent comprising an aqueous dispersion of carboxyalkylalkyl cellulose (JP-A-2000-80048); and a method which comprises adding water to a mixed solution consisting of an organic solvent or a mixed solvent of water and an organic solvent into which a carboxyl group-containing cellulose is dissolved, and a basic substance to precipitate particles (JP-A-2000-186153), etc. have been proposed.

A method wherein fine particles of a water-insoluble polymer are dispersed in water has problems, such as that the suspension must be always stirred because it lacks suspension stability, and that the film-formability is insufficient due to large particle diameters and irregular particle shapes. On the other hand, in the cases of the aqueous dispersions of latex-type in which a part of the carboxyl groups are in the form of salt as in JP-A-5-125224 or JP-A-2000-80048, the particle diameters are small and the dispersed particles are in the form of true spheres, and hence the dispersion stability is very high. The film-formability thereof is, however, insufficient. Thus, when these dispersions are actually used for pharmaceutical enteric preparations, they sometime lack resistance to the first liquid, which is required for an enteric coating agent.

Furthermore, although JP-A-2000-186153 discloses that carboxyl groups in the form of salt are restored to those in the form of acid using an acid after the particle precipitation, the invention disclosed in this publication relates to the production of carboxyl group-containing cellulose in which the particles are purified and dried under a control to give rather larger particle diameters as mentioned hereinafter, and has no idea of obtaining an aqueous dispersion of carboxyl group-containing cellulose with good film-formability.

### DISCLOSURE OF INVENTION

The object of the present invention is to provide a cellulose derivative aqueous dispersion which is high in dispersion stability due to the small particle diameters, as well as, is high in film-formability and resistance to the first liquid.

The resistance to the first liquid will be explained. An enteric pharmaceutical is a preparation that does not release an active ingredients in the stomach, but in the intestines in order to protect the stomach or avoid the decomposition of the active ingredient in the stomach. For not releasing an active ingredient in the stomach, the coating film must not be dissolved in or dispersed in gastric juice. A simple method for evaluation thereof is the disintegration test method prescribed in the Pharmacopeia Japonica (13th Ed.). The resistance to the first liquid means that a film is not dissolved in or dispersed in or a coated preparation releases substantially no active ingredients in the first liquid of pH 1.2 as defined in the above test method.

As a result of intensive researches conducted by the inventors in an attempt to solve the above problems, it has been found that a film excellent in film-formability and resistance to the first liquid can be obtained by converting a part of the carboxyl groups of a cellulose derivative to a salt form by adding an alkali to make the derivative self-emulsification and prepare a pre-treatment aqueous dispersion which is extremely improved in stability of dispersed solid, and then preparing an aqueous dispersion therefrom by removing a part or whole of the alkali. Thus, the present invention has been accomplished.

That is, the present invention relates to the following aspects.
(1) A method of producing a cellulose derivative aqueous dispersion, comprising:
   dissolving a cellulose derivative having a carboxyl group in an organic solvent to obtain a solution;
   dispersing the solution and water in the presence of an alkali in an amount corresponding to 1-65 mol% of the carboxyl groups to obtain a dispersion;
   removing the organic solvent from the dispersion to obtain a pre-treatment aqueous dispersion; and
   removing a part or the whole of the alkali used from the aqueous dispersion.
(2) The method described in the above (1), wherein the organic solvent is an organic solvent immiscible with water.
(3) The method described in the above (1) or (2), wherein a part or the whole of the alkali is removed by adding an acid.
(4) The method described in the above (3), wherein the acid added is an organic acid.
(5) The method described in the above (4), wherein the acid is added in an amount of 6 or less equivalents based on the alkali used.
(6) A cellulose derivative aqueous dispersion obtainable by the method described in any one of the above (1)-(5).
(7) A coated pharmaceutical obtainable by using the cellulose derivative aqueous dispersion described in the above (6) as a film coating material.

### PREFERRED EMBODIMENTS FOR CARRYING OUT THE INVENTION

The present invention will be explained in more detail below.

The cellulose derivatives having carboxyl group(s) suitably usable in the present invention are those which do not dissolve in gastric juice, but in intestinal juice (hereinafter referred to as "enteric cellulose derivatives"). Examples thereof include carboxymethylethyl cellulose, carboxymethylmethyl cellulose, carboxyethylmethyl cellulose, carboxybutylethyl cellulose, carboxyethylethyl cellulose, etc.

Among them, carboxymethylethyl cellulose is preferred as the enteric cellulose derivative used in the present invention, and especially preferred are those which contain 8.9-14.9% of carboxymethyl groups and 32.5-43.0% of ethoxy groups. Since these compounds have no ester bond, they hardly hydrolyze under usual storage conditions and are optimum as materials for a cellulose derivative aqueous dispersion. These may be used either alone or in a suitable combination of two or more. Furthermore, if necessary, these compounds may be used in admixture with polymers having no ionic functional groups, such as ethyl cellulose, or acrylic polymers.

The organic solvents used in the present invention need to dissolve the above enteric cellulose derivatives. As the organic solvents, there may be used, for example, organic solvents which are freely miscible with water, such as methanol, ethanol, acetone, isopropanol and tetrahydrofuran; or organic solvents which are not freely miscible with water, e.g., hydrocarbons such as toluene and cyclohexene, ethers such as isopropyl ether, ketones such as methyl ethyl ketone, halogenated hydrocarbons such as chloroform and methylene chloride, esters such as methyl acetate, ethyl acetate, propyl acetate, isopropyl acetate, methyl formate, ethyl formate and propyl formate.

Here, the organic solvents which are not freely miscible with water include the organic solvents which are utterly immiscible with water and the organic solvents which are not completely miscible with water but which dissolve a little in water or in which water is dissolved a little. As for the organic solvents which are freely miscible with water or partially miscible with water, there can also be used those which contain water, as long as the enteric cellulose derivatives can dissolve thereto.

Since the organic solvents used in the present invention must be removed at the later steps, it is preferred to use those having a boiling point lower than that of water, especially not higher than 90°C. It is advantageous to use organic solvents which are not freely miscible with water because the amount of water to be added will be smaller to cut down a cost for water evaporation and improve productivity, such as working time. Especially preferred are ketones such as methyl ethyl ketone which are not hydrolyzed.

These solvents may be used either alone or as a mixed solvent of two or more to, for example, control the solubility. The concentration of the enteric cellulose derivative dissolved in these solvents is not particularly limited, and can be determined depending on productivity, handling of solution and dispersibility in water. The suitable range of the concentration is preferably about 1-30 wt%, more preferably 4-20 wt%, thought it varies depending on the kind of and the molecular weight of the enteric cellulose derivative and the kind of the solvent.

As to the water used in the present invention, in addition to use of water singly, there may also be used water into which an organic solvent that dissolves a little in water, such as methyl ethyl ketone, methyl acetate or ethyl acetate, or an organic solvent which freely dissolves in water, such as ethanol, is partially dissolved.

In the present invention, the ratio of the solution of the enteric cellulose derivative and water is set depending on the concentration of the enteric cellulose derivative solution and the solid content in the desired pre-treatment aqueous dispersion.

For example, when the concentration of the enteric cellulose derivative solution is 10 wt% and the solid content in the pre-treatment aqueous dispersion is intended to be 20 wt%, the mixing ratio of the solution and water may be set at about 10:4. Of course, one can prepare a pre-treatment aqueous dispersion having lower concentration by dispersion and removal of the solvent with a larger ratio of water than the above ratio and subsequently concentrate it to the desired solid content by removing water by distillation, membrane separation, or the like.

The alkalis used in the present invention are preferably those which dissolve in water to dissociate into monovalent, divalent or trivalent ions and are non-toxic when solid in dispersion is formed. Examples of the alkalis include sodium hydroxide, potassium hydroxide, calcium hydroxide, magnesium hydroxide, aluminum hydroxide, ammonia, sodium carbonate, potassium carbonate, etc. Preferred are those which dissociate into monovalent or divalent ions from the point of stability or viscosity of the pre-treatment aqueous dispersion.

The amount of the alkali varies depending on the kind of and the molecular weight of the cellulose derivative and the kind of the solvent, and is preferably about 0.02-1.3 milliequivalent/amount of cellulose derivative (g). The amount of the alkali in this case corresponds to the amount to neutralize 1-65 mol% of carboxyl groups in the enteric cellulose derivative (in the case of carboxymethylethyl cellulose having a content of carboxymethyl group of 12.0%). Hitherto, it has been very difficult to produce an aqueous dispersion of latex type of polymer by an emulsion solvent removal method without using an emulsifier, but it becomes possible to produce an aqueous dispersion of latex type by utilizing the self-emulsification action of the polymer by adding the alkali in the above-mentioned amount without using an emulsifier.

The amount of alkali added is preferably not more than the amount to neutralize 65 mol% of the carboxyl groups in the enteric cellulose derivative from the viewpoints of viscosity, dispersion stability and film-formability of the aqueous dispersion, and preferably not less than the amount to neutralize 1 mol% from the viewpoints of self-emulsifiability and dispersion stability and film-formability of the aqueous dispersion. It is more preferably 0.12-1.06 milliequivalent/amount of cellulose derivative (g) (this amount of alkali added similarly corresponds to the amount to neutralize 6-53 mol% of carboxyl groups), and further preferably 0.24-0.92 milliequivalent/amount of cellulose derivative (g) (this amount of alkali added similarly corresponds to the amount to neutralize 13-46 mol% of carboxyl groups).

Regarding the method of adding the alkali, it may be mixed in any medium of a mixture of the solution of the enteric cellulose derivative and water, the solution of the enteric cellulose derivative and water, but preferably it is dispersed and dissolved in water so as to avoid local neutralization. Another preferred method is to use an enteric cellulose derivative in which 65 mol% or less of carboxyl groups are previously converted to the salt form, and, if necessary, further add the alkali so that 1-65 mol% of carboxyl groups are in the salt form.

In the present invention, the dispersion of water and the enteric cellulose derivative solution can be carried out either while adding water to the solution of the enteric cellulose derivative or while adding the solution of the enteric cellulose derivative to water. In the case of using an organic solvent which is not freely miscible with water, it is advantageous and preferred from the view points of cost for water evaporation and productivity such as working time to provide an emulsified state of W/O type (water-in-oil type) by decreasing the amount of water added. In the case of using an organic solvent which is freely miscible with water, since the enteric cellulose derivative loses solubility at the stage of mixing the solution and water and is precipitated at this stage, it is preferred to add water gradually so as not to cause precipitation of coarse particles.

Dispersing apparatuses used for dispersing the enteric cellulose derivative solution with water in the presence of an alkali may be those which provide usual stirring force. Examples of the apparatuses include propeller stirrer, homomixer, Marthon Gaulin type homogenizer, Nanomizer (trademark) (Nanomizer Co., Ltd.), Microfluidizer (trademark) (Microfluidics Co.), etc.

When the organic solvent is removed from the resulting dispersion of the enteric cellulose derivative solution with water, true spherical particles of the enteric cellulose derivative are formed in the aqueous phase and thus a pre-treatment aqueous dispersion can be obtained. As aforementioned, when the solid concentration is to be further increased, a part of the water may be removed. The removal of the organic solvent is usually carried out by the method of distillation under normal pressure or reduced pressure, the method of steam stripping, or the method of aeration. Furthermore, when the enteric cellulose derivative solution is dispersed and emulsified with water in the presence of an alkali and successively the organic solvent is continuously removed, it is advantageous to use a vacuum emulsifying machine such as T.K. AGI HOMOMIXER (trademark) (Tokushu Kika Kogyo Co., Ltd.).

When an organic solvent which is not freely miscible with water is used, it is preferred to carry out phase inversion of the emulsion from a W/O type emulsion to an O/W type emulsion to prepare the pre-treatment aqueous dispersion, in order to remove the organic solvent. Although it is naturally possible to produce the pre-treatment aqueous dispersion from an O/W type emulsion without changing the emulsification form, in this case the amount of water added is large and hence water must be removed.

On the other hand, the method of preparing the pre-treatment aqueous dispersion by carrying out removing of the organic solvent with phase inversion is advantageous from the view points of energy efficiency and production efficiency of the apparatuses since the water to be removed can be in the necessary and minimum amount. Hitherto, in case such removal of organic solvent with phase inversion is carried out, emulsification is broken even if the emulsifier is optimized, which makes it difficult to produce a stable pre-treatment aqueous dispersion. However, according to the present invention, it becomes possible to employ such a step because emulsifiability is imparted to the enteric cellulose derivative per se.

The enteric cellulose derivative dispersion of the present invention is obtained by removing a part or whole of the alkali from the pre-treatment aqueous dispersion obtained by removing the organic solvent by the above-mentioned method. Although the pre-treatment aqueous dispersion is small in particle diameter of the dispersed solid and has no problem in dispersion stability, since a resulting film is insufficient in resistance to the first liquid, a large amount of coating is necessary when it is used in pharmaceutical(s) as an enteric coating agent. Particularly, even when there is no problem in resistance to the first liquid in the form of film, the conventional aqueous dispersion is unsatisfactory since the resistance to the first liquid is required under the influences of swelling effect of tablets or granules when used for pharmaceutical(s).

According to the present invention, it has been found that film-formability can be conspicuously improved while maintaining small particle diameter of the dispersed solid, and the resistance to the first liquid of the preparations can be considerably improved. That is, it has become possible to obtain an enteric cellulose derivative aqueous dispersion which has both dispersion stability and film-formability at the same time. It is presumed that this is because carboxyl groups of acid form are higher than those of salt form in compatibility with a plasticizer.

The acids are not particularly limited as long as they are compounds which produce hydrogen ions in water, and there may be used inorganic acids such as, for example, hydrochloric acid, phosphoric acid, sulfuric acid and carbonic acid, and organic acids such as, for example, citric acid, malic acid, tartaric acid, fumaric acid, succinic acid, adipic acid and acetic acid. If an acid with a high degree of dissociation, particularly, a strong acid is used, the acid is localized and a part of the solid in dispersion may be decomposed, and hence a weak acids are more preferred from the point of stability of the cellulose derivative aqueous dispersion, and an organic acid is further preferred, among which citric acid is especially preferred because of its excellent affinity to a plasticizer.

The amount of the acid to be added varies depending on the kind of and the amount of the enteric cellulose derivative and the alkali used and the kind of the acids, etc. In general, when the amount of the alkali to be removed is increased by increasing the amount of the acid added, the solid in dispersion tends to aggregate, and, on the other hand, when the amount of the acid added is insufficient, the resistance to the first liquid of the coating film obtained from the cellulose derivative aqueous dispersion tends to be deteriorated. Therefore, it is preferred to carry out the removal of alkali with using a suitable amount of the acid depending on the kind of the acid.

For example, in the case of using hydrochloric acid, the amount is preferably not less than 0.3 equivalent based on the amount of the alkali added from the view point of the effect to improve film-formability and not more than 1.5 equivalent from the view point of particle diameter when acid and plasticizer are added. It is especially preferred to be 0.6-1 equivalent.

When a weak acid is used, in general, it must be added in a larger amount as compared with the strong acid. When an organic acid is added, the amount of the acid is preferably not more than 6 equivalents, more preferably 0.6-6 equivalents, most preferably 0.6-4 equivalents based on the amount of the alkali used. When using citric acid, the amount is also preferably not more than 6 equivalents, more preferably 0.6-6 equivalents, most preferably 0.6-4 equivalents based on the amount of the alkali used.

The ion exchange resin may be in the form of particles such as of sulfonic acid type and carboxylic acid type which are generally used, but those of weakly acidic carboxylic acid type are preferred because they hardly cause aggregation of the solid in dispersion. They can be directly introduced into the pre-treatment aqueous dispersion, which is then stirred and left. The ion exchange resin can be then removed by filtration.

The median diameter of the dispersed solid particles in the enteric cellulose derivative aqueous dispersion is about 0.05-3 µm, and the maximum diameter is preferably not more than 10 µm. From the viewpoints of dispersibility, film-formability and resistance to the first liquid, the median diameter is preferably not more than 3 µm, and the maximum diameter is preferably not more than 10 µm. The smaller the diameter of the solid particles, the higher the dispersion stability and the film-formability become, nevertheless, from the viewpoint of viscosity, the median diameter is preferably not less than 0.05 µm. Especially, it is preferred to be 0.08-2 µm, and more preferred to be 0.08-1 µm.

The shape of the dispersed solid particles becomes nearly true spheres. The particles in the shape of true spheres result in superior dispersion stability and higher packing of particles at the time of film formation, and thus the film-formability is improved.

The solid concentration of the cellulose derivative aqueous dispersion is preferably not less than 5 wt% from the viewpoint of the time required for film coating and not more than 40 wt% from the viewpoint of coating workability. Especially, it is preferred to be 10-30 wt%.

In order to further improve the physical properties of the cellulose derivative aqueous dispersion and the coating film or to facilitate the production of the pre-treatment aqueous dispersion, additives such as a plasticizer, an anti-foaming agent and a suspending agent may be added at the time of dispersion or addition of acid or to the resulting cellulose derivative aqueous dispersion.

As the plasticizer, citrates, such as triethyl citrate and acetylated triethyl citrate; diacetin, triacetin, olive oil, peanut oil, castor oil, hard fats, glycerin; glycerin fatty acid esters such as glycerin caprylate and acetylated monoglyceride; butylphthalylbutyl glycolate, polyethylene glycol, propylene glycol, sucrose fatty acid esters, medium chain fatty acid esters, sebacates, phthalates, cetanol, D-sorbitol, etc. are included.

Examples of the anti-foaming agent are silicone resin emulsion, silicone anti-foaming agent, dimethylpolysiloxane, etc. Examples of the suspending agent are sodium alginate, carboxymethyl cellulose, hydroxypropyl cellulose, hydroxypropylmethyl cellulose, polyvinyl alcohol, polyvinyl pyrrolidone, polyethylene glycol, methyl cellulose, etc. These additives may be used alone or as a mixture of two or more.

Particularly, it is preferred to add the plasticizer for improving the film-formability. The amount of the plasticizer to be added is preferably not less than 5 wt% based on the solid content of the enteric cellulose derivative from the viewpoints of plasticizing effect, film-formability and resistance to the first liquid, and not more than 100 wt% from the viewpoint of coating property and from the viewpoint of resistance to the first liquid in the case of hydrophilic plasticizer. Especially preferably, it is 15-80 wt%. It is further preferably 25-60 wt%.

Although the use of the emulsifier may cause adverse effects on the physical properties of the film, such as change of solubility with time, if necessary, sodium laurylsulfate, polyoxyethylenenonylphenyl ether, polyoxyethylenesorbitan monooleate, polyoxysorbitan tristearate, etc. may be used.

The enteric cellulose derivative aqueous dispersion, optionally with addition of various additives such as a plasticizer, when used for coating of tablets, granules, fine granules, etc. by known methods, can provide an excellent enteric coated pharmaceutical(s). Because it is an aqueous solid in dispersion, coating can be performed with high concentrations without increasing the viscosity not so much upon coating, and hence the coating time can be short. Furthermore, because of superior film-formability, the enteric preparation can be obtained with a small coating amount.

The coating amount in a pharmaceutical(s) varies depending on the size of the pharmaceutical(s), other additives and apparatuses used, but is preferably 3-50 wt% based on the pharmaceuticals before coating. In the case of tablets, about 3-15 wt% is preferred, in the case of granules, about 5-25 wt% is preferred, and in the case of fine granules, about 10-50 wt% is preferred.

As coating apparatuses, those which are usually employed can be used, and for tablets, apparatuses for film coating of tablets such as coating pan, HICOATER (FREUND INDUSTRIAL Co., Ltd.), etc. can be employed. For granules and fine granules, for example, fluidized bed coating apparatus, rotating fluidized bed coating apparatus, centrifugal fluidized bed type granulation coating apparatus, etc. are included. As to the operating conditions, there may be selected those which are suitable for the respective apparatus.

### Examples

The present invention will be explained in detail by the following examples, which should not be construed as limiting the invention in any manner. The methods of measuring various physical properties employed in the following examples and the comparative examples are as shown below.

### (Particle size distribution)

A measurement was conducted using a laser type particle size distribution measuring apparatus LA-910 (manufactured by Horiba Ltd.) at a refractive index of 1.20, and a median diameter at 50% cumulative volume was obtained.

### (Tablet disintegration test)

### (a) Disintegration test with the first liquid

A the test was conducted on six coated tablets using a disintegration tester (manufactured by Toyama Sangyo Co., Ltd.) in accordance with a disintegration test method for enteric tablets prescribed in the Pharmacopeia Japonica, the 13th Ed. The test fluid used was the first liquid having a temperature of 37°C.

### (b) Disintegration test with the second liquid

The test was conducted in the same manner as above, except that the second liquid having a temperature of 37°C was used as the test fluid.

### (Granule dissolution test)

### (a) Release test with the first liquid

A test was conducted on coated granules using an dissolution test machine (manufactured by JASCO Co.) in accordance with a release test (paddle method with 100 rpm) mentioned in the Pharmacopeia Japonica, 13th Ed. As a test fluid, 900 ml of the first liquid having a temperature of 37°C was used.

### (b) Dissolution test with the second liquid

The test was conducted in the same manner as above, except that the second liquid having a temperature of 37°C was used as a test fluid.

### (Example 1)

Eighty grams of carboxymethylethyl cellulose (CMEC, OS manufactured by FREUND INDUSTRIAL Co., Ltd. and containing 12.0% of carboxymethyl groups and 39.0% of ethoxyl groups) was dissolved in 730 g of methyl ethyl ketone saturated with water to prepare a solution. Then, the solution was stirred at 12000 rpm for 30 minutes by a homomixer while adding thereto a solution prepared by dispersing and dissolving 1.6 g of sodium hydroxide (which was 0.50 milliequivalent based on 1 g of carboxymethylethyl cellulose and corresponded to the amount by which 25 mol% of carboxylic acid is formed into salt) in 190 g of water saturated with methyl ethyl ketone, thereby performing emulsification. The resulting emulsion was of water-in-oil type, and the continuous phase was a solution of carboxymethylethyl cellulose in methyl ethyl ketone.

The emulsion was charged in a three-necked flask and methyl ethyl ketone was removed at 40°C and under 13 kPa while stirring by a three-one motor. Therein the phase inversion occurred between the continuous phase and the dispersion phase in about 1 hour, and the aqueous phase became the continuous phase. After adding 120 g of water, distillation was further carried out for 4 hours to obtain a stable pre-treatment aqueous dispersion (a) with a solid content of 20%, wherein carboxymethylethyl cellulose was dispersed in water. This aqueous dispersion (a) had a median diameter of 0.12 µm.

Ten grams of triethyl citrate was added to 100 g of the aqueous dispersion (a) while stirring at 4000 rpm by a homomixer. Then 55 g of a 2% aqueous citric acid solution (which was 0.85 milliequivalent based on 1 g of carboxymethylethyl cellulose and corresponded to 1.7 equivalent based on 1 equivalent of the alkali added) was further added thereto to obtain a cellulose derivative aqueous dispersion (A). The aqueous dispersion (A) had a median diameter of 0.13 µm.

The aqueous dispersion (A) was coated on an aluminum plate and dried at 80°C for 1 hour to form a transparent and gloss coating film, which was very good. This coating film was dipped in the first liquid and subjected to an ultrasonic treatment for 1 minute. However, no sign of dispersion of the coating film was observed and thus the coating film showed very high resistance to the first liquid. The results are shown in Table 1.

### (Example 2)

Fifty-five grams of a 2% aqueous citric acid solution was added to the aqueous dispersion (a) obtained in Example 1 while stirring at 4000 rpm by a homomixer, followed by adding 10 g of triethyl citrate to obtain a cellulose derivative aqueous dispersion (B). The aqueous dispersion (B) had a median diameter of 0.13 µm.

The film-formability and the resistance to the first liquid of the aqueous dispersion (B) were evaluated in the same manner as in Example 1. The film-formability and the resistance to the first liquid were equal to those obtained in Example 1. The results are shown in Table 1.

### (Example 3)

A pre-treatment aqueous dispersion (c) was obtained in the same manner as in Example 1, except that the amount of sodium hydroxide was changed to 0.8 g (which was 0.25 milliequivalent based on 1 g of carboxymethylethyl cellulose and corresponded to the amount by which 12 mol% of carboxylic acid is formed into salt). The aqueous dispersion (c) had a median diameter of 0.41 µm. A cellulose derivative aqueous dispersion (C) was obtained in the same manner as in Example 1, except that the amount of the 2% aqueous citric acid solution added to the above aqueous dispersion (c) was changed to 19 g (which was 0.30 milliequivalent based on 1 g of carboxymethylethyl cellulose and corresponded to 1.2 equivalent based on the equivalent of the alkali added). The aqueous dispersion (C) had a median diameter of 0.43 µm.

The film-formability and the resistance to the first liquid of the aqueous dispersion (C) were evaluated in the same manner as in Example 1. The film-formability and the resistance to the first liquid were equal to those obtained in Example 1. The results are shown in Table 1.

### (Example 4)

A pre-treatment aqueous dispersion (d) was obtained in the same manner as in Example 1, except that the amount of sodium hydroxide was changed to 0.4 g (which was 0.13 milliequivalent based on 1 g of carboxymethylethyl cellulose and corresponded to the amount by which 6 mol% of carboxylic acid is formed into salt). The aqueous dispersion (d) had a median diameter of 0.80 µm. A cellulose derivative aqueous dispersion (D) was obtained in the same manner as in Example 1, except that the amount of 2% aqueous citric acid solution added to the above aqueous dispersion (d) was changed to 15 g (which was 0.24 milliequivalent based on 1 g of carboxymethylethyl cellulose and corresponded to 1.9 equivalent based on the equivalent of the alkali added). The aqueous dispersion (D) had a median diameter of 0.85 µm.

The film-formability and the resistance to the first liquid of the aqueous dispersion (D) were evaluated in the same manner as in Example 1. The film-formability and the resistance to the first liquid were equal to those obtained in Example 1. The results are shown in Table 1.

### (Example 5)

A pre-treatment aqueous dispersion (e) was obtained in the same manner as in Example 1, except that 2.2 g of calcium hydroxide (which was 0.74 milliequivalent based on 1 g of carboxymethylethyl cellulose and corresponded to the amount by which 37 mol% of carboxylic acid is formed into salt) was used in place of sodium hydroxide. The aqueous dispersion (e) had a median diameter of 0.33 µm. A cellulose derivative aqueous dispersion (E) was obtained in the same manner as in Example 1, except that the amount of 2% aqueous citric acid solution added to the above aqueous dispersion was changed to 78 g (which was 1.21 milliequivalent based on 1 g of carboxymethylethyl cellulose and corresponded to 1.6 equivalent based on the equivalent of the alkali added). The aqueous dispersion (E) had a median diameter of 0.33 µm.

The film-formability and the resistance to the first liquid of the aqueous dispersion (E) were evaluated in the same manner as in Example 1. The film-formability and the resistance to the first liquid were equal to those obtained in Example 1. The results are shown in Table 1.

### (Example 6)

A cellulose derivative aqueous dispersion (F) was obtained in the same manner as in Example 1, except that in place of citric acid, 58 g of 0.5% hydrochloric acid (which was 0.40 milliequivalent based on 1 g of carboxymethylethyl cellulose and corresponded to 0.8 equivalent based on the equivalent of the alkali added) was added to 100 g of the aqueous dispersion (a) and that 7 g of triacetin was added in place of triethyl citrate. The aqueous dispersion (F) had a median diameter of 0.48 µm.

The film-formability and the resistance to the first liquid of the aqueous dispersion (F) were evaluated in the same manner as in Example 1. As a result, the coating film formed was equal to the coating film obtained in Example 1, although the coating film was somewhat inferior in gloss to the latter. Furthermore, no dispersion of the coating film was observed in the test on resistance to the first liquid, but the coating film was somewhat hazed as compared with the coating film of Example 1. The results are shown in Table 1.

### (Example 7)

Forty grams of carboxymethylethyl cellulose used in Example 1 was dissolved in 360 g of methyl acetate saturated with water to prepare a solution. Then, the solution was stirred at 12000 rpm for 30 minutes by a homomixer while adding thereto a solution prepared by dispersing and dissolving 2 g of an anti-foaming agent (silicone resin preparation KM72A manufactured by Shin-Etsu Chemical Co., Ltd.) and 1.1 g of potassium hydroxide (which was 0.50 milliequivalent based on 1 g of carboxymethylethyl cellulose and corresponded to the amount by which 25 mol% of carboxylic acid is formed into salt) in 95 g of water saturated with methyl acetate, thereby to perform emulsification. The resulting emulsion was of water-in-oil type, and the continuous phase was a solution of carboxymethylethyl cellulose in ethyl acetate.

The emulsion was charged in a three-necked flask, and methyl acetate was removed at 40°C under 13 kPa while stirring by a three-one motor. Therein, the phase inversion occurred between the continuous phase and the dispersion phase in about 1 hour, and the aqueous phase became the continuous phase. After adding 100 g of water, removal was further carried out for 4 hours to obtain a stable pre-treatment aqueous dispersion (g) with a solid content of 15% wherein carboxymethylethyl cellulose was dispersed in water. This aqueous dispersion (g) had a median diameter of 0.09 µm.

Seven grams of glyceryl caprylate was added to 100 g of the aqueous dispersion (g) while stirring at 4000 rpm by a homomixer, followed by adding 42 g of a 2% aqueous citric acid solution (which was 0.85 milliequivalent based on 1 g of carboxymethylethyl cellulose and corresponded to 1.7 equivalent based on the equivalent of the alkali added) to obtain a cellulose derivative aqueous dispersion (G). The aqueous dispersion (G) had a median diameter of 0.09 µm.

The film-formability and the resistance to the first liquid of the aqueous dispersion (G) were evaluated in the same manner as in Example 1. The film-formability and resistance to the first liquid were equal to those obtained in Example 1. The results are shown in Table 1.

### (Example 8)

To 100 g of the aqueous dispersion (a) of Example 1 was added 12 g of a carboxylic acid-type ion exchange resin IRC76 (manufactured by Organo Co., Ltd.), followed by stirring for 2 hours and then being left to stand for one night. Then, the ion exchange resin was removed by filtration, and to the resulting solution was added 7 g of triethyl citrate while stirring by a homomixer to obtain a cellulose derivative aqueous dispersion (H). The aqueous dispersion (H) had a median diameter of 0.60 µm.

The film-formability and the resistance to the first liquid of the aqueous dispersion (H) were evaluated in the same manner as in Example 1. As a result, the coating film formed was equal to the coating film obtained in Example 1, although the coating film was somewhat inferior in gloss to the latter. Furthermore, no dispersion of the coating film was observed in the test of the resistance to the first liquid, but the coating film was somewhat hazed as compared with the coating film obtained in Example 1. The results are shown in Table 1.

### (Example 9)

Sixty grams of carboxymethylethyl cellulose used in Example 1 was dissolved in 140 g of ethanol and 60 g of water to prepare a solution. Then, 2 g of sodium hydroxide (50% in concentration) (which was 0.42 milliequivalent based on 1 g of carboxymethylethyl cellulose and corresponded to the amount by which 20 mol% of carboxylic acid is formed into salt) was added to the solution while stirring at 12000 rpm by a homomixer, followed by stirring for 10 minutes. Then, while similarly stirring, 400 g of water was added dropwise thereto over a period of 90 minutes, followed by further stirring for 2 hours to precipitate cellulose derivative particles. The resulting dispersion was charged in a three-necked flask, and ethanol and water were removed at 40°C under 13 kPa while stirring by a three-one motor to obtain a pre-treatment aqueous dispersion (i) of 20% in solid concentration. This aqueous dispersion (i) had a median diameter of 0.52 µm.

Seven grams of glyceryl caprylate was added to 100 g of the aqueous dispersion (i) while stirring at 4000 rpm by a homomixer, followed by further adding 49 g of a 2% aqueous citric acid solution (which was 0.61 milliequivalent based on 1 g of carboxymethylethyl cellulose and corresponded to 1.5 equivalent based on the equivalent of the alkali added) to obtain a cellulose derivative aqueous dispersion (I). The aqueous dispersion (I) had a median diameter of 0.50 µm.

The film-formability and the resistance to the first liquid of the aqueous dispersion (I) were evaluated in the same manner as in Example 1. As a result, the coating film formed was similar to the coating film obtained in Example 1, although the former was somewhat inferior in gloss to the latter. Furthermore, no dispersion of the coating film was observed in the test on the resistance to the first liquid, but the coating film was somewhat hazed as compared with the coating film obtained in Example 1. The results are shown in Table 1.

### (Example 10)

A pre-treatment aqueous dispersion (j) was obtained in the same manner as in Example 9, except that the amount of sodium hydroxide (50% in concentration) was changed to 1 g (which was 0.21 milliequivalent based on 1 g of carboxymethylethyl cellulose and corresponded to the amount by which 10 mol% of carboxylic acid is formed into salt). The aqueous dispersion (j) had a median diameter of 0.97 µ m. A cellulose derivative aqueous dispersion (J) was obtained in the same manner as in Example 9, except that 25 g of a 2% aqueous citric acid solution (which was 0.30 milliequivalent based on 1 g of carboxymethylethyl cellulose and corresponded to 1.4 equivalent based on the equivalent of the alkali added) was used for 100 g of the aqueous dispersion (j). The aqueous dispersion (J) had a median diameter of 0.96 µ m.

The film-formability and the resistance to the first liquid of the aqueous dispersion (J) were evaluated in the same manner as in Example 1. As a result, the coating film formed was equal to the coating film obtained in Example 1, although the coating film was somewhat inferior to the latter in transparent feeling and gloss. Furthermore, no dispersion of the coating film was observed in the test on the resistance to the first liquid, but the coating film was somewhat hazed as compared with the coating film obtained in Example 1. The results are shown in Table 1.

### (Example 11)

A cellulose derivative aqueous dispersion (K) was obtained in the same manner as in Example 1, except that the amount of the 2% aqueous citric acid solution added to 100 g of the aqueous dispersion (a) of Example 1 was changed to 117 g (which was 1.80 milliequivalent based on 1 g of carboxymethylethyl cellulose and corresponded to 3.6 equivalents based on the equivalent of the alkali added). The aqueous dispersion (K) had a median diameter of 0.20 µm.

The film-formability and the resistance to the first liquid of the aqueous dispersion (K) were evaluated in the same manner as in Example 1. The film-formability and the resistance to the first liquid were equal to those obtained in Example 1. The results are shown in Table 1.

### (Example 12)

A cellulose derivative aqueous dispersion (L) was obtained in the same manner as in Example 1, except that the amount of the 2% aqueous citric acid solution added to 100 g of the aqueous dispersion (a) of Example 1 was changed to 140 g (which was 2.13 milliequivalents based on 1 g of carboxymethylethyl cellulose and corresponded to 4.3 equivalents based on the equivalent of the alkali added). The aqueous dispersion (L) had a median diameter of 0.34 µm.

The film-formability and the resistance to the first liquid of the aqueous dispersion (L) were evaluated in the same manner as in Example 1. As a result, the coating film obtained was equal to the coating film obtained in Example 1. Further, no dispersion of the coating film was observed in the test of the resistance to the first liquid, but the coating film was somewhat hazed as compared with the coat obtained in Example 1. The results are shown in Table 1.

### (Example 13)

A cellulose derivative aqueous dispersion (M) was obtained in the same manner as in Example 1, except that 51 g of a 2% aqueous succinic acid solution (which was 0.5 milliequivalent based on 1 g of carboxymethylethyl cellulose and corresponded to 1.7 equivalent based on the equivalent of the alkali added) was added to 100 g of the aqueous dispersion (a) of Example 1. The aqueous dispersion (M) had a median diameter of 0.17 µm.

The film-formability and the resistance to the first liquid of the aqueous dispersion (M) were evaluated in the same manner as in Example 1. The film-formability and the resistance to the first liquid were similar to those obtained in Example 1. The results are shown in Table 1.

### (Example 14)

Micro-crystalline cellulose "AVICEL®" PH-101 (manufactured by Asahi Kasei Co.)/100 mesh lactose (manufactured by DMV Co., Ltd.)/Aspirin (manufactured by Hoei Yakko Co., Ltd.)/magnesium stearate (manufactured by Taihei Kagaku Co., Ltd.) were mixed at a weight ratio of 30/30/40/0.5. The mixture was then formed into tablets having a diameter of 8 mm, a weight of 250 mg and a tablet hardness of 9 kg using a rotary tablet machine.

Four hundred grams of the tablets were charged in a film coating apparatus for tablets, HICOATER MINI (manufactured by FREUND INDUSTRIAL Co., Ltd.), and the coating of the tablets was carried out under the conditions of a vessel rotating speed of 10 rpm and an air temperature of 80°C, by spraying 2 g/min of each of the aqueous dispersions (A), (C)-(J) and (M) so that the amount of the coat reached 5% based on the weight of the tablet. Subsequently, the tablets were treated by a hot-air drying machine at 80°C for 1 hour to obtain enteric coated tablets.

Six of each coated tablet were subjected to the disintegration test with the first liquid for 2 hours, but no damage to the coating film, such as disintegration, was observed. Separately, six of the each tablet were subjected to the disintegration test with the second liquid, and it was found that the tablets were all disintegrated within 1 hour. Thus, all of the coated tablets functioned as enteric tablets.

### (Example 15)

Five hundred grams of seed granules comprising nucleus particles Celphere® CP-305 (manufactured by Asahi Kasei Co.) on the surface of which 2 wt% of riboflavin (manufactured by Mitsubishi Tokyo Seiyaku Co., Ltd.) was layered were charged in a granule coating apparatus MULTIPLEX MP-01 (manufactured by Powrex Co., Ltd.), and the granules were coated with the aqueous dispersion (A) until the coating amount reached 20 wt% based on the seed granules by spraying the aqueous dispersion (A) at a rate of 13 g/min under the conditions of a revolution number of rotating plate of 400 rpm and an air supply temperature of 65°C. Successively, the granules were subjected to pre-drying at 40°C for 30 minutes by a hot-air drying machine and then treated at 80°C for 1 hour to obtain enteric coated granules.

The coated granules were subjected to the release test with the first liquid for 2 hours. As a result, substantially no release was seen.
Furthermore, a separate release test with the second liquid on the coated granules showed rapid dissolution. Thus, the coated granules functioned as enteric granules.

### (Comparative Example 1)

The same procedure as Example 1 was carried out, except that sodium hydroxide was not used in the preparation of the aqueous dispersion (a). Therein, a separation of the aqueous phase occurred and the median diameter was large, namely, 8 µm, and good aqueous dispersion was not obtained.

The coating film test on the resulting aqueous dispersion in the same manner as in Example 1 gave no good coating film. The results are shown in Table 2.

### (Comparative Example 2)

The same procedure of Example 1 was performed, except that the amount of sodium hydroxide was changed to 4.5 g (which was 1.4 milliequivalent based on 1 g of carboxymethylethyl cellulose and corresponded to the amount by which 70 mol% of carboxylic acid is formed into salt). The dispersion thus obtained was a viscous white gel, and good aqueous dispersion was not obtained. The aqueous dispersion had a median diameter of 7 µm.

The coating film test on this aqueous dispersion in the same manner as in Example 1 gave no good coating film. The results are shown in Table 2.

### (Comparative Example 3)

Ten grams of triethyl citrate was added to 100 g of the aqueous dispersion (a) obtained in Example 1 while stirring at 4000 rpm by a homomixer to obtain an aqueous dispersion (N) (a method according to JP-A-5-125224). The aqueous dispersion (N) had a median diameter of 0.12 µm.

The coating film test on the aqueous dispersion (N) in the same manner as in Example 1 gave a nearly equal coating film. This coating film was, however, observed to partially disperse after being dipped in the first liquid and subjected to ultrasonic treatment for 1 minute. The coating film was thus inferior in the resistance to the first liquid. The results are shown in Table 2.

### (Comparative Example 4)

The same procedure as Example 9 was performed, except that sodium hydroxide was not used. Therein, a separation of the aqueous phase occurred and the median diameter was large, namely, 5 µm, and a good aqueous dispersion was not obtained. The coating film test on the resulting aqueous dispersion gave no good coating film. The results are shown in Table 2.

### (Comparative Example 5)

The same procedure as Example 9 was carried out, except that citric acid was not added, to obtain an aqueous dispersion (O) (a method according to JP-A-2000-80048). The aqueous dispersion (O) had a median diameter of 0.54 µm.

The coating film test on the aqueous dispersion (O) in the same manner as in Example 1 gave a nearly equal coating film, which was somewhat inferior in gloss though. The coating film was, however, observed to partially disperse after being dipped in the first liquid and subjected to ultrasonic treatment for 1 minute. The coating film was thus inferior in the resistance to the first liquid. The results are shown in Table 2.

### (Comparative Example 6)

The same procedure as of Example 14 was carried out using the aqueous composition of Comparative Example 3 to obtain coated tablets. The disintegration test with the first liquid on these coated tablets in the same manner as in Example 14 resulted in that five tables among six were disintegrated within 2 hours.

### (Comparative Example 7)

The same procedure as of Example 14 was carried out using the aqueous composition of Comparative Example 5 to obtain coated tablets. The disintegration test with the first liquid on these coated tablets in the same manner as in Example 14 resulted in that six tablets were all disintegrated within 2 hours.

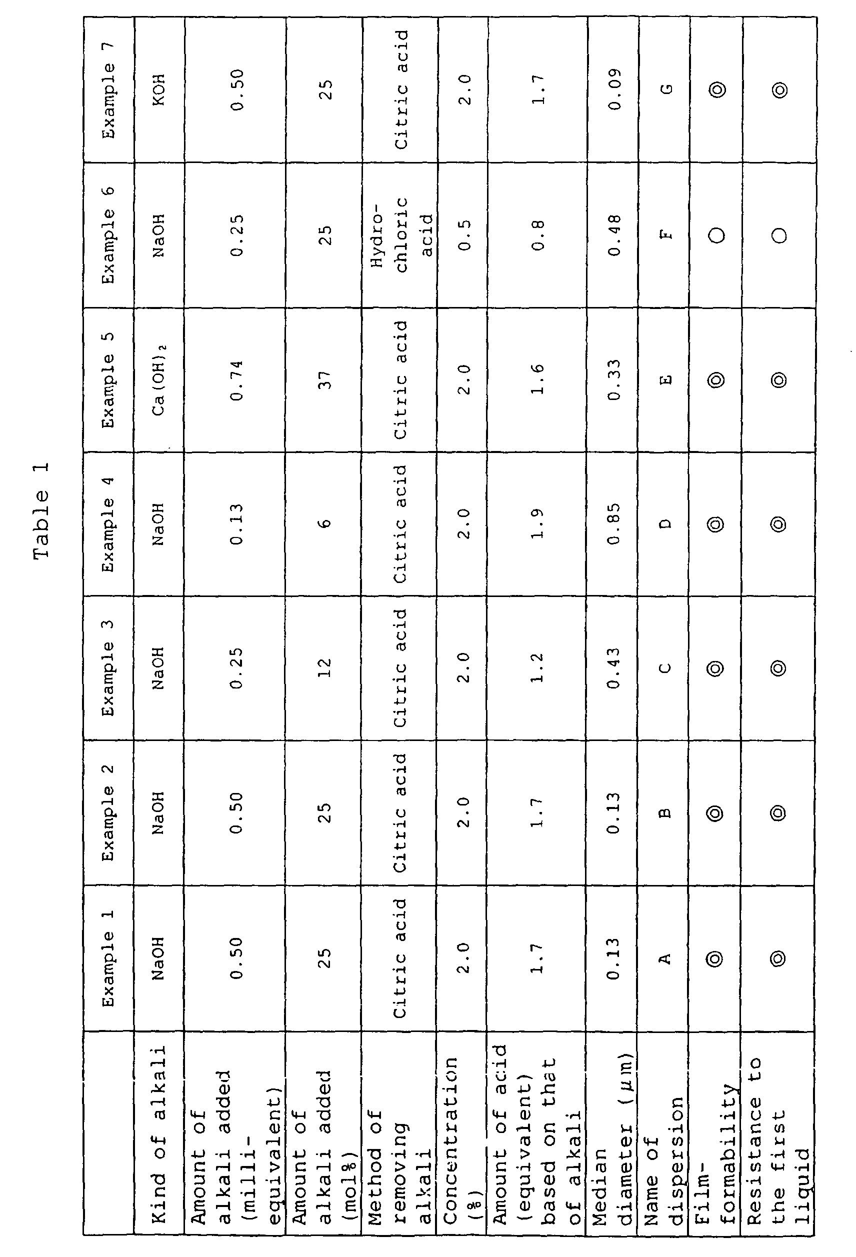

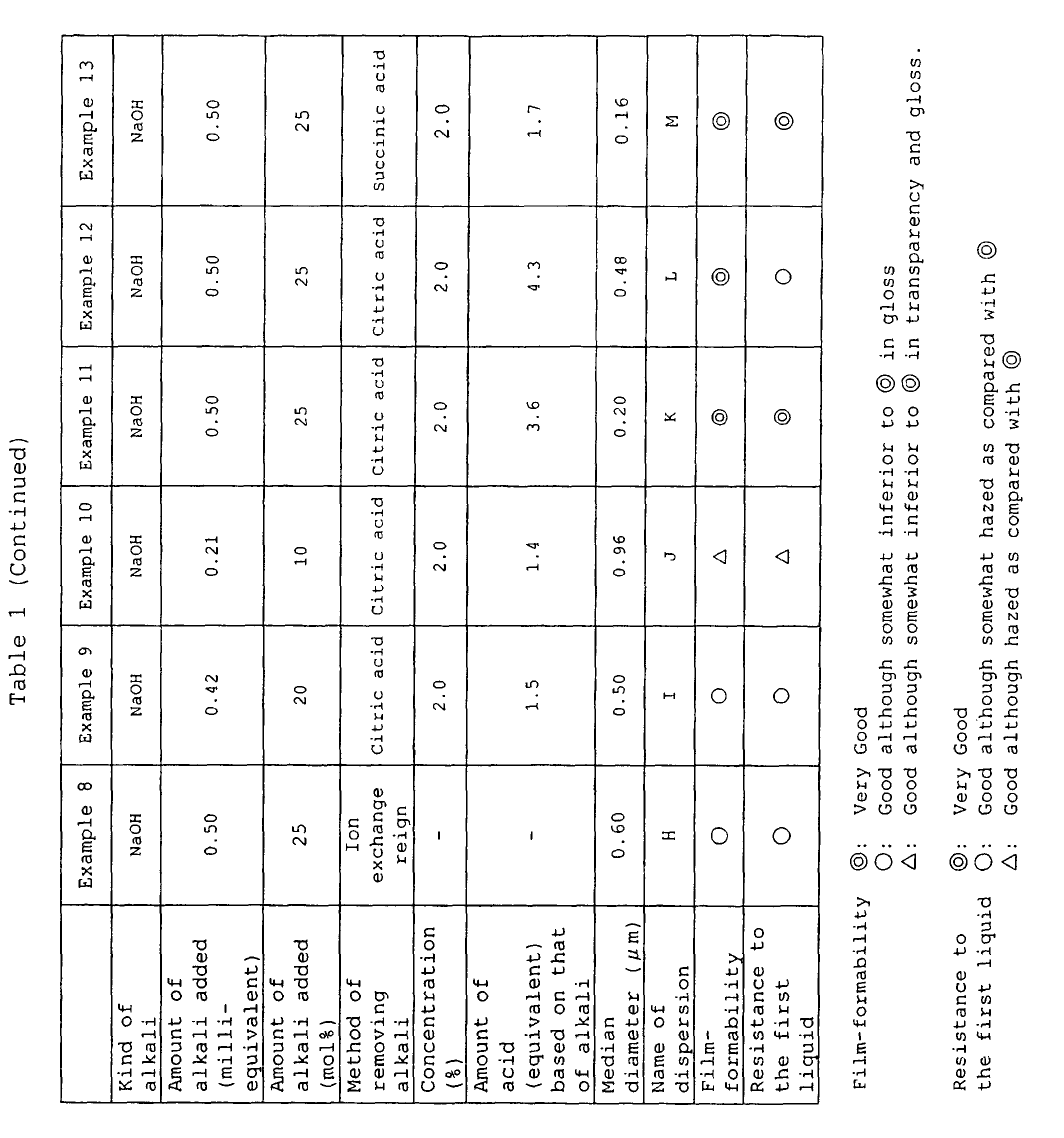

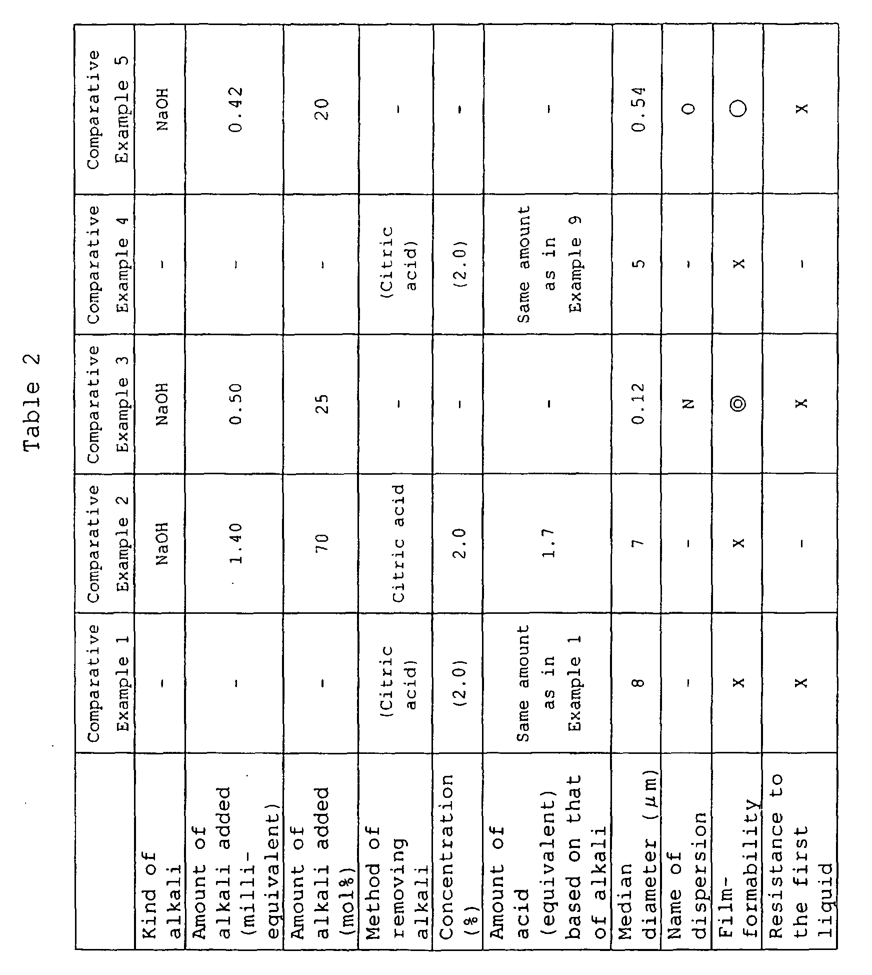

### INDUSTRIAL APPLICABILITY

The enteric cellulose derivative aqueous dispersions obtained according to the present invention are excellent in dispersion stability and resistance to the first liquid. Therefore, when they are used as a coating agent for pharmaceutical(s), the enteric properties are superior as compared with conventional enteric cellulose derivative aqueous latex.

## Claims

1. A method of producing a cellulose derivative aqueous dispersion, comprising:
dissolving a cellulose derivative having a carboxyl group in an organic solvent to obtain a solution;
dispersing the solution and water in the presence of an alkali in an amount corresponding to 1-65 mol% of the carboxyl groups to obtain a dispersion;
removing the organic solvent from the dispersion to obtain a pre-treatment aqueous dispersion; and
removing a part or the whole of the alkali from the aqueous dispersion.

2. The method according to claim 1, wherein the organic solvent is an organic solvent immiscible with water.

3. The method according to claim 1 or 2, wherein a part or the whole of the alkali is removed by adding an acid.

4. The method according to claim 3, wherein the acid added is an organic acid.

5. The method according to claim 4, wherein the acid is added in an amount of 6 or less equivalents based on the alkali used.

6. A cellulose derivative aqueous dispersion obtainable by the method according to any one of claims 1-5.

7. A coated pharmaceutical obtainable by using the cellulose derivative aqueous dispersion according to claim 6 as a film coating material.

## Patentansprüche

1. Verfahren zur Herstellung einer wässrigen Dispersion eines Cellulosederivats, umfassend:
Auflösen eines Cellulosederivats, das eine Carboxygruppe aufweist, in einem organischen Lösungsmittel, so dass man eine Lösung erhält;
Dispergieren der Lösung und Wasser in Gegenwart eines Alkali in einer Menge, die 1-65 Mol-% der Carboxygruppen entspricht, so dass man eine Dispersion erhält;
Entfernen des organischen Lösungsmittels aus der Dispersion, so dass man eine wässrige Vorbehandlungsdispersion erhält; und
Entfernen eines Teils oder des gesamten Alkali aus der wässrigen Dispersion.

2. Verfahren gemäß Anspruch 1, wobei das organische Lösungsmittel ein mit Wasser nicht mischbares organisches Lösungsmittel ist.

3. Verfahren gemäß Anspruch 1 oder 2, wobei ein Teil oder das gesamte Alkali durch Zugabe einer Säure entfernt wird.

4. Verfahren gemäß Anspruch 3, wobei die hinzugefügte Säure eine organische Säure ist.

5. Verfahren gemäß Anspruch 4, wobei die Säure in einer Menge von 6 oder weniger Äquivalenten, bezogen auf das verwendete Alkali, zugegeben wird.

6. Wässrige Dispersion eines Cellulosederivats, die nach dem Verfahren gemäß einem der Ansprüche 1 bis 5 erhältlich ist.

7. Beschichtetes Pharmakon, das unter Verwendung der wässrigen Dispersion eines Cellulosederivats gemäß Anspruch 6 als Filmbeschichtungsmaterial erhältlich ist.

## Revendications

1. Procédé de production d'une dispersion aqueuse d'un dérivé Cellulosique, comprenant :
de dissoudre un dérive cellulosique ayant un groupe carboxyle dans un solvant organique pour obtenir une solution ;
de disperser la solution et de l'eau en présence d'un alcali en quantité correspondant à 1-65 mol% des groupes carboxyles pour obtenir une dispersion ;
d'enlever le solvant organique de la dispersion pour obtenir une dispersion aqueuse de prétraitement ; et
d'enlever une partie ou la totalité de l'alcali de la dispersion aqueuse.

2. Procédé selon la revendication 1, dans lequel le solvant organique est un solvant organique non miscible avec l'eau.

3. Procédé selon la revendication 1 ou 2, dans lequel une partie ou la totalité de l'alcali est enlevée en ajoutant un acide.

4. Procédé selon la revendication 3, dans lequel l'acide ajouté est un acide organique.

5. Procédé selon la revendication 4, dans lequel l'acide est ajouté en quantité inférieure ou égale à 6 équivalents sur la base de l'alcaü utilisé.

6. Dispersion aqueuse d'un dérivé cellulosique qu'on peut obtenir par le procédé selon l'une quelconque des revendications 1-5.

7. Produit pharmaceutique enrobé qu'on peut obtenir en utilisant la dispersion aqueuse d'un dérivé cellulosique selon la revendication 6 comme matière d'enrobage par un film.
